# EUROPÄISCHE PATENTSCHRIFT

(11) **EP 0 382 078 B1**
(45) Veröffentlichungstag und Bekanntmachung des Hinweises auf die Patenterteilung: **22.09.1993**
(21) Anmeldenummer: 90101948.9
(22) Anmeldetag: 01.02.1990
(51) Int. Cl.: C07C 65/36, C07C 65/40, C07C 69/76, C07C 69/92, A61K 31/235, A61K 31/19, A61K 7/48

(54) **Aromatische Ketoverbindungen, ihre Herstellung und Arzneimittel sowie Kosmetika daraus**
Aromatic keto compounds, their preparation and medicinal as well as cosmetic preparations containing them
Composés céto-aromatiques, leur préparation et médicaments ainsi que cosmétiques les contenant

(30) Priorität: 10.02.1989 DE 3903992
(43) Veröffentlichungstag der Anmeldung: 16.08.1990
(73) Patentinhaber: BASF Aktiengesellschaft, 67063 Ludwigshafen (DE)
(72) Erfinder: Wuest, Hans-Heiner, Dr., D-6915 Dossenheim (DE); Janssen, Bernd, Dr., D-6700 Ludwigshafen (DE)

(56) Entgegenhaltungen:
- EP-A- 0 237 986
- EP-A- 0 260 162
- JOURNAL OF MEDICINAL CHEMISTRY, Band 32, Nr. 5, Mai 1989, Seiten 1098-1108, American Chemical Society, Washington, US; K. SHUDO et al.: "Retinobenzoic acids. 3. Structure-activity relationships of retinoidal azobenzene-4-carboxylic acids and stilbene-4-carboxylic acids"

## Beschreibung

Die Erfindung betrifft neue aromatische Ketoverbindungen, Verfahren zu deren Herstellung sowie daraus hergestellte Mittel zur Bekämpfung und Prophylaxe von Krankheiten.

Aus den DE-OS 2 854 354 und DE-OS 3 202 118 ist bekannt, daß retinoidale Benzoesäurederivate pharmakologische Wirkungen bei der topischen und systemischen Therapie von Neoplasien und Dermatosen, z.B. Akne oder Psoriasis aufweisen. Nachteil dieser Verbindungen ist ihre geringe therapeutische Breite bezüglich der unter dem Begriff Hypervitaminose A zusammengefaßten Nebenwirkungen. EP-A-237 986 beschreibt Flavorderivate mit ähnlicher Wirkung.

Es wurde nun gefunden, daß Verbindungen der allgemeinen Formel I 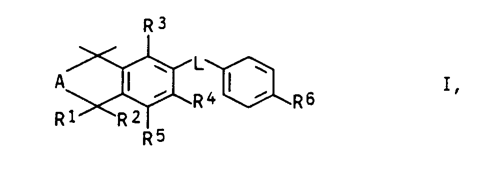
in der
A einen gegebenenfalls mit einer Methyl-, Hydroxyl- oder Oxogruppe substituierten Ethylen- oder Methylenrest,
L eine der Gruppen 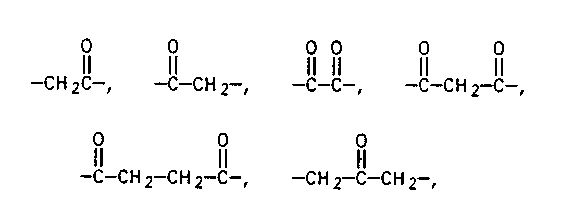
R¹ und R² Wasserstoff oder einen Methylrest,
R³ Wasserstoff, eine Hydroxy- oder eine C₁₋₆-Alkoxygruppe,
R⁴ Wasserstoff, einen C₁₋₄-Alkylrest, ein Halogenatom oder eine Methoxygruppe,
R⁵ Wasserstoff oder eine Methoxygruppe und
R⁶ Wasserstoff, eine Methyl- oder Nitrilgruppe, eine C₂₋₁₀-Ketalgruppe der Formel 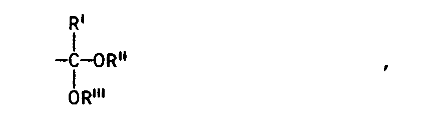
wobei R′, R˝, und R‴ Alkylgruppen mit 1 bis 9 C-Atomen darstellen, die Summe der C-Atome von R′, R˝ und R‴ 2 bis 10 beträgt und R′ auch Wasserstoff sein kann,
oder die Reste
-CHR⁷-OR⁸, -CHR⁷-NR⁹R¹⁰, -COR¹¹, 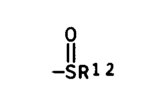
oder -SO₂R¹¹
bedeuten, worin
R⁷ Wasserstoff oder eine C₁₋₄-Alkylgruppe,
R⁸ Wasserstoff, eine C₁₋₄-Alkyl-, C₁₋₂₀-Alkanoyl-, oder eine gegebenenfalls substituierte Benzoylgruppe,
R⁹ und R¹⁰ Wasserstoffatome, C₁₋₄-Alkyl-, C₁₋₆-Alkanoyl- oder gegebenenfalls substituierte Benzoylgruppen oder zusammen mit dem Stickstoffatom, an das sie gebunden sind, einen 5- oder 6-gliedrigen, gesättigten heterocyclischen Rest, der außer dem Stickstoffatom auch zusätzlich ein Sauerstoffatom enthalten kann,
R¹¹ Wasserstoff, eine C₁₋₄-Alkylgruppe oder die Reste -OR¹³ oder -NR¹⁴R¹⁵ mit R¹³ in der Bedeutung eines Wasserstoffatoms, einer gegebenenfalls durch 1 bis 2 Hydroxylgruppen substituierten C₁₋₈-Alkylgruppe, einer gegebenenfalls substituierten Aryl- oder gegebenenfalls im Arylteil substituierten Aralkylgruppe, und mit R¹⁴ und R¹⁵ in der Bedeutung von Wasserstoffatomen, gegebenenfalls substituierten Aralkyl- oder Arylgruppen, oder
R¹⁴ und R¹⁵ zusammen mit dem Stickstoffatom, an das sie gebunden sind, in der Bedeutung eines 5- oder 6-gliedrigen gesättigten heterocyclischen Restes, der außer dem Stickstoffatom auch zusätzlich ein Sauerstoffatom enthalten kann, darstellen, und
R¹² eine C₁₋₄-Alkylgruppe bedeutet,
sowie deren physiologisch verträgliche Salze ein verbessertes Wirkprofil, vor allem hinsichtlich der Nebenwirkungen, besitzen.

Als heterocyclische Reste -NR⁹R¹⁰ und -NR¹⁴R¹⁵ kommen insbesondere Pyrrolidinyl, Piperidinyl und Morpholinyl in Betracht. Bevorzugte Substituenten der Benzoylgruppe (R⁸, R⁹, R¹⁰) sind die Methoxy-, Nitro- oder Methylgruppen sowie Halogenatome, insbesondere Chlor, Brom. Bevorzugt als Arylrest (R¹³, R¹⁴, R¹⁵) ist die Phenylgruppe, die durch eine Methyl-, Methoxy- oder Nitrilgruppe substituiert sein kann. Als Aralkylgruppe (R¹³, R¹⁴, R¹⁵) ist die Benzylgruppe bevorzugt, die im Arylteil insbesondere durch eine Methyl- oder Methoxygruppe oder ein Halogenatom, vorzugsweise Chlor oder Brom, substituiert sein kann.

Ist R⁴ ein Halogenatom, so ist Fluor bevorzugt.

Die erfindungsgemäßen Verbindungen der Formel I lassen sich nach im Prinzip bekannten Verfahren herstellen. Eine Übersicht über die verschiedenen Herstellungsmethoden von Ketonen findet sich in "Methoden der Organischen Chemie" (Houben-Weyl-Müller), Band 7/2a-c, Georg Thieme Verlag Stuttgart.

Die im Wesentlichen für die Synthese der erfindungsgemäßen Verbindungen verwendeten Methoden seien im folgenden beschrieben:
1. Desoxybenzoine der Formel I (L = -CH₂-CO- oder -CO-CH₂-) kann man beispielsweise erhalten, indem man
   a) einen Phosphonsäureester der Formel II 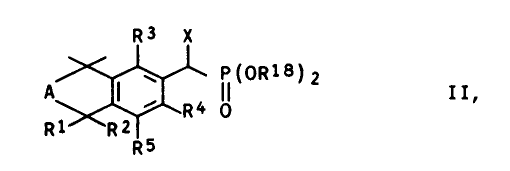 worin A und R¹-R⁵ die obengenannten Bedeutungen haben und X einen Rest -OR¹⁶ oder NR¹⁶R¹⁷ mit R¹⁶ und R¹⁷ in der Bedeutung eines C₁₋₄-Alkylrestes, wobei die Reste R³ und R¹⁶ zusammen eine C₁₋₃-Alkylenkette bilden können, und R¹⁸ einen C₁₋₄-Alkylrest bedeutet,
      mit einem Aldehyd der Formel III 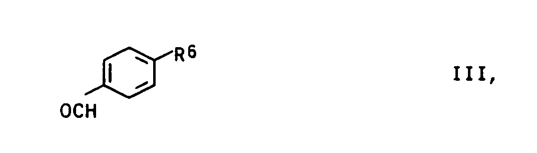 in der R⁶ die oben genannte Bedeutung hat,
      in Gegenwart einer Base im Sinne einer Wittig-Horner-Reaktion umsetzt und das daraus erhaltene Enamin (X = NR¹⁶R¹⁷) bzw. den Enolether (X = OR¹⁶) sauer hydrolysiert, oder indem man
   b) ein Carbonsäurechlorid der Formel IV bzw. V 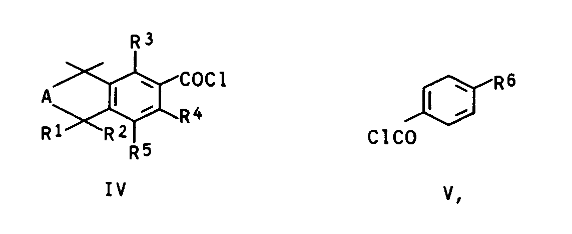 in denen A und R¹-R⁶ die oben genannten Bedeutungen haben, mit einem Benzylhalogenid der Formel VI bzw. VII 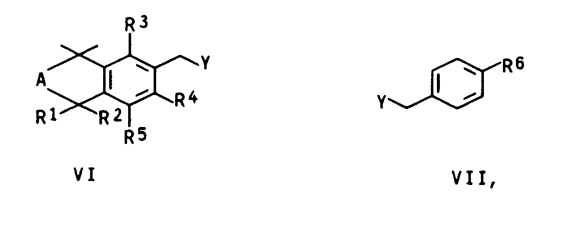 in denen A und R¹-R⁶ die oben genannten Bedeutungen haben, und Y für Chlor oder Brom steht,
      in Gegenwart eines geeigneten Metalls, gegebenenfalls unter Zusatz katalytischer Mengen eines Übergangsmetallkatalysators, umsetzt, wobei entweder IV mit VII oder V mit VI umgesetzt wird.
2. Erfindungsgemäße 1,3-Diketoverbindungen der Formel I mit L = -C(O)CH₂C(O)- bzw. deren enoltautomere Verbindungen lassen sich beispielsweise herstellen, indem man
   a) ein Keton der Formel VIII 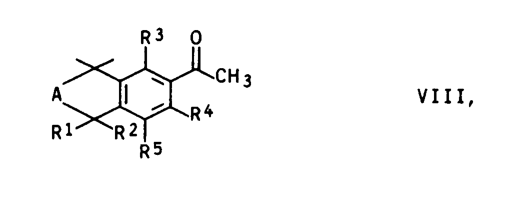 in der A und R¹-R⁵ die oben genannten Bedeutungen haben, mit einem Terephthalsäureester der Formel IX 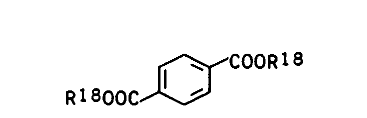 in der R¹⁸ für eine C₁₋₄-Alkylgruppe steht,
      in Gegenwart einer Base umsetzt, oder indem man
   b) an ein Chalkon der Formel X 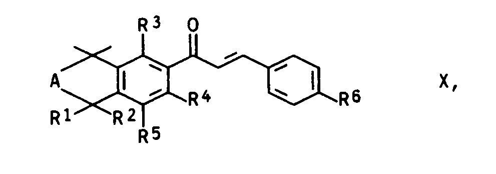 in der A und R¹-R⁶ die oben genannten Bedeutungen haben,
      zunächst Brom addiert, dann mit einer geeigneten Base Bromwasserstoff eliminiert und schließlich sauer hydrolysiert.
3. Die erfindungsgemäßen 1,4-Diketoverbindungen der Formel I (L = -C(O)CH₂CH₂C(O)-) lassen sich herstellen, indem man ein Chlorethylketon der Formel XI 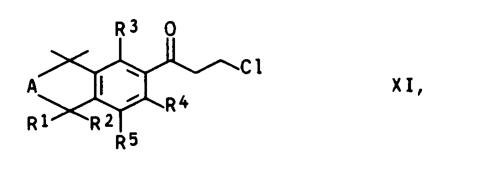 in der A und R¹-R⁵ die oben genannten Bedeutungen haben,
   mit einer geeigneten Base zunächst zu einem Vinylketon der Formel XII 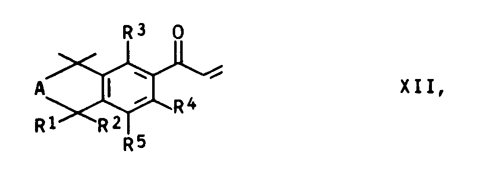 in der A und R¹-R⁵ die oben genannten Bedeutungen haben, umsetzt, und dieses mit einem Benzaldehyd der Formel XIII 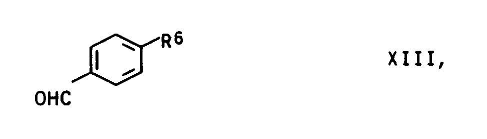 in der R⁶ die oben genannte Bedeutung hat,
   in Gegenwart eines "Umpolungs"-Katalysators weiter umsetzt (s. z.B. H. Stetter und J. Krasselt, J. Heterocyclic Chem. 14, 573 (1977).
4. Ferner kann man die nach den unter 1. - 3. beschriebenen oder nach anderen Verfahren hergestellten Verbindungen der Formel I durch Abwandlung des Restes R⁶ in weitere erfindungsgemäße Verbindungen der Formel I überführen.
   Die Wittig-Horner-Reaktion gemäß 1.a) wird in den dafür üblichen Lösungsmitteln wie Tetrahydrofuran, Diethylether, 1,2-Dimethoxyethan, n-Hexan, Petrolether, Toluol, Dimethylsulfoxid, Dimethylformamid oder Gemischen daraus bei Temperaturen von -78 - +60°C durchgeführt, wobei die Temperatur stark von der eingesetzten Base abhängig gemacht wird.

Als Basen finden Natriumhydrid, das Natriumsalz von Dimethylsulfoxid, Alkoholate wie Natriummethanolat oder Kalium-tert.butanolat, oder Lithiumorganyle wie n-Butyllithium Verwendung. Bevorzugt verwendet man zur Metallierung der Phosphonsäureester der Formel II n-Butyllithium als Base bei einer Temperatur zwischen -78 und -60°C, gibt danach die Benzaldehydkomponente zu und läßt bei einer Temperatur bis 25°C fertigreagieren.

Die Umsetzung gemäß 1.b) erfordert mindestens äquimolekulare Mengen eines Metalls und/oder Metallsalzes. Als Zwischenprodukte treten die entsprechenden metallorganischen Verbindungen auf, so z.B. des Magnesiums, Cadmiums, Zinks oder Lithiums. Besonders vorteilhaft ist Zink, vorzugsweise mit Kupfer oder Silber vorher aktiviertes Zink. Gegebenenfalls setzt man katalytische Mengen eines Übergangsmetallkatalysators zu, vorzugsweise des Palladiums und Nickels, z.B. Bis(triphenylphosphin)palladium(II)-chlorid oder Tetrakis(triphenylphosphin)-palladium. Der Zusatz solcher Katalysatoren ist besonders günstig bei der Verwendung von Zink als reduktivem Metall.

Als Lösungsmittel verwendet man vor allem Ether wie Diethylether, Tetrahydrofuran, Dioxan oder 1,2-Dimethoxyethan, eventuell auch aromatische Kohlenwasserstoffe wie Benzol oder Toluol. Die Umsetzungen werden bei einer Temperatur zwischen 0 und 60°C, vorzugsweise bei 20 - 40°C durchgeführt.

Esterkondensationen gemäß 2.a) können prinzipiell unter basischer als auch saurer Katalyse stattfinden, meistens finden jedoch Basen Verwendung, z.B. Alkalimetallhydride wie Natriumhydrid, Alkalimetallalkoholate wie Natriummethanolat, Natriumethanolat, Kalium-tert.-butanolat oder Alkalimetallamide wie Lithium-, Natrium-oder Kaliumamid.

Zweckmäßigerweise verwendete Lösungsmittel sind Alkohole wie Methanol oder Ethanol, Ether wie Diethylether, Diisopropylether, Tetrahydrofuran, Dioxan oder 1,2-Dimethoxyethan, aromatische Kohlenwasserstoffe wie Toluol oder Xylol, oder auch Dimethylformamid. Die Umsetzungen werden bei einer Temperatur zwischen 20 und 140°C, vorzugsweise zwischen 80 und 120°C durchgeführt.

Geeignete Basen gemäß 2.b) sind z.B. Alkoholate wie Natriummethanolat oder Kalium-tert.-butanolat. Die Hydrolyse wird mit verdünnten oder konzentrierten Mineralsäuren wie Salzsäure oder Schwefelsäure bewerkstelligt.

Zur Eliminierung von Chlorwasserstoff gemäß 3. sind vor allem organische Stickstoffbasen wie Triethylamin oder Pyridin geeignet. Die Vinylketone der Formel XII können isoliert werden, günstig ist jedoch die weitere Umsetzung in situ. Die Kupplung mit dem Benzaldehydderivat der Formel XIII im Sinne einer Umpolungsreaktion wird durch Cyanidanionen oder noch besser durch Thiazoliumsalze wie 3-Benzyl-5-(2-hydroxyethyl)-4-methylthiazoliumchlorid katalysiert. Als Lösungsmittel verwendet man bevorzugt Dimethylformamid.

Zu den Methoden gemäß 4. gehören beispielsweise:
Benzoesäureester oder Benzonitrile der allgemeinen Formel I, in der R⁶ eine Carboalkoxy- bzw. eine Nitrilgruppe bedeutet, lassen sich in die freien Carbonsäuren und ihre physiologisch verträglichen Salze durch Verseifung überführen. Die Verseifung wird vorzugsweise in einem Gemisch eines niederen aliphatischen Alkohols wie Methanol, Ethanol, Propanol, Isopropanol oder n-Butanol mit Wasser in Gegenwart eines im Überschuß eingesetzten Alkalihydroxids, vorzugsweise Natrium- oder Kaliumhydroxids beim Siedepunkt des Reaktionsgemisches durchgeführt.

Die erfindungsgemäßen Amide können in an sich bekannter Weise hergestellt werden, indem man die entsprechenden Benzoesäuren zunächst in carbonylaktivere Derivate überführt, z.B. in die Säurehalogenide, -azide, -imidazolide oder -anhydride, und diese mit Aminen HNR¹⁴R¹⁵ behandelt.

Eine Carbonsäure, ein Carbonsäureester, ein Carbonsäureamid oder ein Nitril der Formel I kann in an sich bekannter Weise zu den entsprechenden Alkoholen bzw. Aminen reduziert werden. Vorteilhaft wird die Reaktion mit Hilfe eines Metallhydrids oder Alkalimetallhydrids in Gegenwart eines geeigneten Lösungsmittels durchgeführt. Als Metallhydride werden vorzugsweise komplexe Metallhydride wie Lithiumaluminium- oder Lithiumborhydrid oder Diisobutylaluminiumhydrid eingesetzt. Bevorzugte Lösungsmittel sind Ether wie Diethylether, Tetrahydrofuran, Dioxan oder 1,2-Dimethoxyethan.

Ein Alkohol oder Amin der Formel I kann in an sich bekannter Weise mit einem Alkanoyl- oder Aroylchlorid oder -anhydrid zu dem entsprechenden Ester bzw. Amid acyliert werden, oder mit einem Alkylhalogenid, vorzugsweise einem Alkylbromid oder -iodid, zu dem entsprechenden Ether oder höheralkylierten Amin alkyliert werden, oder mit geeigneten Oxidationsmitteln, wie Mangan(IV)-oxid, zu dem entsprechenden Aldehyd oxidiert werden.

Ein Aldehyd der Formel I kann auch durch Reduktion des entsprechenden Nitrils der Formel I mit Diisobutylaluminiumhydrid in einem Lösungsmittel, vorzugsweise Toluol, Hexan oder Tetrahydrofuran, in einem Temperaturbereich zwischen -40°C und Raumtemperatur erhalten werden.

Die erfindungsgemäßen Verbindungen und ihre physiologisch verträglichen Salze können aufgrund ihrer pharmakologischen Eigenschaften bei der topischen und systemischen Therapie und auch Propylaxe von Praekanzerosen und Karzinomen der Haut, der Schleimhäute und inneren Organe sowie bei der topischen und systemischen Therapie von Akne, Psoriasis und anderer mit pathologisch veränderter Verhornung einhergehenden dermatologischen Erkrankungen, insbesondere Ichthyosis, Dariersche Krankheit, Flechte, Leukoplakie, aber auch gegen Ekzeme, Vitiligo, Warzen, Lichtschäden (vorzeitige Alterung) der Haut, ferner gegen trockene Augen und andere Corneopathien sowie zur Behandlung von rheumatischen Erkrankungen, insbesondere solcher entzündlicher oder degenerativer Art, die Gelenke, Muskeln, Sehnen und andere Teile des Bewegungsapparates befallen, verwendet werden. Ein bevorzugtes Indikationsgebiet ist neben der Therapie von dermatologischen Erkrankungen und durch Sonnenlichteinwirkung oder iatrogen, z.B. durch Corticosteroide induzierte Atrophie, bedingten Hautschädigungen die Prophylaxe gegen Praekanzerosen und Tumoren.

Die pharmakologischen Wirkungen können beispielsweise in den nachfolgenden Testmodellen aufgezeigt werden: Die erfindungsgemäßen Verbindungen heben an Hamstertrachealgewebe in vitro die nach Vitamin-A-Mangel einsetzende Keratinisierung auf. Die Keratinisierung gehört zur Frühphase der Carcinogenese, die in einer ähnlichen Technik in vivo nach der Initiation durch chemische Verbindungen, durch energetische Strahlung oder nach viraler Zelltransformation durch die erfindungsgemäßen Verbindungen der Formel I inhibiert wird. Diese Methodik kann aus Cancer Res. 36, 964-972 (1972) oder aus Nature 250, 64-66 (1074) und Nature 253, 47-50 (1975) entnommen werden.

Darüber hinaus wird durch die erfindungsgemäßen Verbindungen die Proliferation bestimmter maligne veränderter Zellen inhibiert. Diese Methodik kann aus J. Natl. Cancer Inst. 60, 1035-1041 (1978), Experimental Cell Research 117, 15-22 (1978) und Proc. Natl. Acad. Sci. USA 77, 2937-2940 (1980) entnommen werden.

Die antiarthritische Wirkung der erfindungsgemäßen Verbindungen kann in üblicher Weise im Tierexperiment im Adjuvans-Arthritis- oder Streptokokkenzellwandinduzierten-Arthritis-Modell bestimmt werden. Die dermatologische Aktivität, beispielsweise für die Behandlung von Akne, kann u.a. durch die komedolytische Aktivität und die Fähigkeit nachgewiesen werden, die Anzahl der Zysten im Modell der Rhino-Maus zu reduzieren.

Diese Methode ist von L.H. Kligman et al, in The Journal of Investigative Dermatology 73, 354-358 (1978) beschrieben.

Als weiteres Maß für die dermatologische Aktivität kann die Reduktion der Talgdrüsen und die damit einhergehende verminderte Talgproduktion am Seitenorgan des Hamsters dienen. Diese Methodik ist von E.C. Gomez in J. Am. Dermatol. 6, 746-750 (1982) beschrieben.

Ferner kann die durch die erfindungsgemäßen Verbindungen erzielbare Reversion von Hautschäden, welche durch UV-Licht ausgelöst werden, in Tiermodellen bestimmt werden. Diese Methode ist von L.H. Kligman et al. beschrieben in Connect. Tissue Res. 12, 139-150 (1984) und in J. Am. Acad. Dermatol. 15, 779-785 (1986).

Dementsprechend sind ein weiterer Gegenstand der Erfindung therapeutische Mittel zur topischen und systemischen Anwendung sowie kosmetische Mittel, die eine Verbindung der Formel I neben üblichen Trägerstoffen oder Verdünnungsmitteln als Wirkstoff enthalten.

Die Mittel können peroral, parenteral oder topisch verabreicht werden. Derartige Zubereitungen sind beispielsweise Tabletten, Filmtabletten, Dragees, Kapseln, Pillen, Pulver, Lösungen oder Suspensionen, Infusions- oder Injektionslösungen sowie Pasten, Salben, Gele, Cremes, Lotionen, Puder, Lösungen oder Emulsionen und Sprays.

Die therapeutischen und kosmetischen Mittel können die erfindungsgemäß zu verwendenden Verbindungen bei lokaler Anwendung in 0,0001 bis 1 %iger Konzentration, bevorzugt in 0,001 bis 0,1 %iger Konzentration, und bei systemischer Anwendung vorzugsweise in einer Einzeldosis von 0,1 bis 50 mg enthalten und täglich in einer oder mehreren Dosierungen je nach Art und Schwere der Erkrankungen verabreicht werden.

Die Arzneimittel und Kosmetika der Erfindung werden mit den üblichen festen oder flüssigen Trägerstoffen oder Verdünnungsmitteln und den üblicherweise verwendeten pharmazeutisch-technischen Hilfsstoffen entsprechend der gewünschten Applikationsart mit einer geeigneten Dosierung in bekannter Weise hergestellt. Tabletten können beispielsweise durch Mischen des Wirkstoffs mit bekannten Hilfsstoffen, beispielsweise inerten Verdünnungsmitteln wie Dextrose, Zucker, Sorbit, Mannit, Polyvinylpyrrolidon, Sprengmitteln wie Maisstärke oder Alginsäure, Bindemitteln wie Stärke oder Gelatine, Gleitmitteln wie Magnesiumstearat oder Talk und/oder Mitteln zur Erzielung eines Depoteffektes wie Carboxypolymethylen, Carboxymethylcellulose, Celluloseacetatphthalat oder Polyvinylacetat, erhalten werden. Die Tabletten können auch aus mehreren Schichten bestehen.

Entsprechend können Dragees durch Überziehen von analog den Tabletten hergestellten Kernen mit üblicherweise in Drageeüberzügen verwendeten Mitteln, beispielsweise Polyvinylpyrrolidon oder Schellack, Gummiarabicum, Talk, Titandioxid oder Zucker, hergestellt werden. Dabei kann auch die Drageehülle aus mehreren Schichten bestehen, wobei die oben bei den Tabletten erwähnten Hilfsstoffe verwendet werden können.

Lösungen oder Suspensionen mit dem erfindungsgemäßen Wirkstoff können zusätzlich geschmacksverbessernde Mittel wie Saccharin, Cyclamat oder Zucker sowie z.B. Aromastoffe wie Vanillin oder Orangenextrakt, enthalten. Sie können außerdem Suspendierhilfsstoffe wie Natriumcarboxymethylcellulose oder Konservierungsstoffe wie p-Hydroxybenzoate, enthalten. Wirkstoffe enthaltende Kapseln können beispielsweise hergestellt werden, indem man den Wirkstoff mit einem inerten Träger, wie Milchzucker oder Sorbit, mischt und in Gelatinekapseln einkapselt.

Zweckmäßige übliche Bestandteile von kosmetischen und pharmazeutischen Zubereitungen für die topische Anwendung sind beispielsweise:
anionische, kationische sowie nichtionische Emulgatoren und Emulsionsstabilisatoren, die gleichzeitig Konsistenzgeber oder Gelbildner sein können, wie Polyvinylpyrrolidon, Fettalkohole, Glycerinmonostearat, Polyacrylsäuren, Cellulosederivate und Ethylenoxid-Propylenoxid-Blockpolymere,
feste oder flüssige Ölkomponenten bzw. Fettstoffe mineralischer, pflanzlicher oder tierischer Herkunft, synthetische Esteröle, wie Glyceroltriester und Isopropylmyristat,
hydrophile Komponenten, wie Glycerin, Polyethylenglykol und Propylenglykol.

Weiterhin können als Inhaltsstoffe von Kosmetika genannt werden beispielsweise Lichtschutzmittel, Bräunungsmittel, Konservierungsmittel, Antioxidantien, Pigmente, Farbstoffe, etherische Öle und Parfümöle, Vitamine, Pflanzenextrakte, Collagen usw. Diese Stoffe können beispielsweise dem CTFA, Cosmetic Ingredient Dictionary, 3. Auflage, Washington 1982, entnommen werden.

Einige der erfindungsgemäßen Verbindungen weisen ein acides Wasserstoffatom auf und können daher mit Basen in üblicher Weise in ein physiologisch verträgliches, gut wasserlösliches Salz übergeführt werden. Geeignete Salze sind beispielsweise Ammonium-, Alkalimetallsalze, insbesondere des Natriums, Kaliums und Lithiums, und Erdalkalimetallsalze, insbesondere des Calciums oder Magnesiums, sowie Salze mit geeigneten organischen Basen, wie C₁₋₆-Alkylaminen, z.B. Methylamin, Ethylamin oder Cyclohexylamin, oder mit substituierten C₁₋₆-Alkylaminen, insbesondere hydroxysubstituierten Alkylaminen, wie Diethanolamin, Triethanolamin und Tris(hydroxymethyl)aminomethan, sowie mit Piperidin oder Morpholin.

Gegebenenfalls werden die erhaltenen erfindungsgemäßen Amine der Formel I nach bekannter Verfahrensweise in das Säureadditionssalz einer physiologisch verträglichen Säure übergeführt. Als übliche physiologisch verträgliche anorganische Säuren kommen beispielsweise in Betracht Salzsäure, Bromwasserstoffsäure, Phosphorsäure oder Schwefelsäure und als organische Säuren beispielsweise Oxalsäure, Maleinsäure, Fumarsäure, Milchsäure, Weinsäure, Äpfelsäure, Zitronensäure, Salicylsäure, Adipinsäure oder Benzoesäure. Weitere können aus "Fortschritte der Arzneimittelforschung" Band 10, Seiten 224-225, Birkhäuser Verlag, Basel und Stuttgart, 1966, entnommen werden.

### Beispiel 1

### 1-(4-Carbomethoxyphenyl)-3-(5,6,7, 8-tetrahydro-5,5,8,8-tetramethyl-2-naphthyl)-1,3-propandion

Zu einer Suspension von 3,6 g (0,15 mol) Natriumhydrid in 21 ml Toluol wurde unter Stickstoff bei 100°C eine Lösung von 19,4 g (0,1 mol) Terephthalsäuredimethylester und 23 g (0,1 mol) 6-Acetyl-1,2,3,4-tetrahydro-1,1,4,4-tetramethylnaphthalin in 80 ml Toluol und 20 ml Dimethoxyethan zugetropft. Man ließ 5 h unter Rückfluß rühren. Nach dem Abkühlen wurde das Reaktionsgemisch mit 50 ml Wasser versetzt, mit halbkonzentrierter Salzsäure angesäuert, auf 500 ml Wasser gegossen und mit Chloroform extrahiert. Ausgefallenes Produkt wurde abgesaugt, die Chloroform phase über Natriumsulfat getrocknet und eingeengt. Rückstand und Rohprodukt wurden zusammengegeben und mehrmals mit heißem Methanol gewaschen. Nach Trocknen des Kristallisats erhielt man 22,1 g der Titelverbindung, Schmp. 128°C.

### Beispiel 2

### 1-(4-Carbomethoxyphenyl)-3-(2,3-dihydro-1,1,2,3,3-pentamethyl-5-(1H)-indenyl-1,3-proandion

Analog Beispiel 1 wurden aus 23 g (0,1 mol) 5-Acetyl-2,3-dihydro-1,1,2,3,3-pentamethyl-(1H)-inden und 19,4 g (0,1 mol) Terephthalsäuredimethylester 18,1 g der Titelverbindung erhalten, Schmp. 120-125°C.

### Beispiel 3

### 1-(4-Carbomethoxyphenyl)-3-(5,6,7,8-tetrahydro-3,8,8-trimethyl-2-naphthalenyl)-1,3-propandion

Analog Beispiel 1 wurden aus 10 g (46 mmol) 7-Acetyl-1,2,3,4-tetrahydro-1,1,6-trimethylnaphthalin und 9 g (46 mmol) Terephthalsäuredimethylester 4,8 g der Titelverbindung erhalten, Schmp. 91-92°C.

### Beispiel 4

### 1-(4-Carboxyphenyl)-3-(5,6,7,8-tetrahydro-5,5,8,8-tetramethyl-2-naphthalenyl)-1,3-propandion

20 g (87 mmol) 6-Acetyl-1,2,3,4-tetrahydro-1,1,4,4-tetramethylnaphthalin, 13 g (87 mmol) 4-Formylbenzoesäure und 8 g Natriumhydroxydplätzchen wurden in 100 ml Methanol über Nacht bei Raumtemp. gerührt. Danach goß man auf Eis/Wasser und extrahierte mit Ether. Etherreste in der wäßrigen Phase wurden durch Durchblasen von Stickstoff entfernt. Man säuerte mit konz. Salzsäure an, saugte das ausgefallene Kristallisat ab und erhielt nach Trocknen 24,9 g 3-(4-Carboxyphenyl)-1-(5,6,7,8-tetrahydro-5,5,8,8-tetramethyl-2-naphthalenyl)-2-propen-1-on, Schmp. 199-201°C.

Zu einer Lösung von 18,1 g (50 mmol) dieser Chalkoncarbonsäure in 100 ml Methylenchlorid tropfte man bei -5 bis 0°C eine Lösung von 8,5 g (53 mmol) Brom in 100 ml Methylenchlorid. Man ließ 1 h nachrühren und zog dann das Lösungsmittel im Vakuum ab.

Der Rückstand wurde in 85 ml Methanol aufgenommen und zu 29,2 g einer 30 %igen methanolischen Natriummethanolatlösung gegeben. Das Reaktionsgemisch wurde 3 h bei 65°C gerührt. Man ließ auf 25°C abkühlen und säuerte mit 9 ml konz. Salzsäure an. Es wurde nochmals 3 h bei 65°C gerührt und dann über Nacht bei Raumtemp. stehengelassen. Der ausgefallene Niederschlag wurde abgesaugt und getrocknet.

Zur Reinigung wurde das Rohprodukt in 2 N Natronlauge gelöst und mit Ether 3 x extrahiert. Restlicher Ether wurde aus der wäßrigen Phase mit Stickstoff ausgetrieben, dann säuerte man mit konz. Salzsäure an. Nach Absaugen und Trocknen des Kristallisats erhielt man 10,3 g der Titelverbindung, Schmp. 200-202°C.

### Beispiel 5

### 1-(4-Carbomethoxyphenyl)-4-(5,6,7,8-tetrahydro-5,5,8,8-tetra-methyl-2-naphthalenyl)-1,4-butandion

Zu einer Lösung von 70 g (0,55 mol) Chlorpropionsäurechlorid in 200 ml Methylenchlorid wurden bei 0 bis 5°C 73,5 g (0,55 mol) wasserfreies Aluminiumchlorid portionsweise zugegeben und danach bei derselben Temperatur eine Lösung von 94 g (0,5 mol) 1,2,3,4-Tetrahydro-1,1,4,4-tetramethylnaphthalin in 200 ml Methylenchlorid zugetropft. Man ließ über Nacht bei Raumtemperatur rühren, goß auf 1 l Eis/Wasser und extrahierte 3 x mit je 300 ml Methylenchlorid. Die vereinigten organischen Phasen wurden mit ges. Natriumhydrogencarbonatlösung und Wasser gewaschen, über Magnesiumsulfat getrocknet und eingeengt. Es verblieben 138 g 6-(3-Chlorpropionyl)-1,2,3,4-tetrahydro-1,1,4,4-tetramethylnaph-thalin als Öl. Die Struktur wurde durch H-NMR-Spektroskopie abgesichert.

5,6 g (0,02 mol) 6-(3-Chlorpropionyl)-1,2,3,4-tetrahydro-1,1,4,4-tetramethylnaphthalin und 3,3 ml (0,024 mol) Triethylamin wurden in 30 ml Dimethylformamid 1 h bei Raumtemp. gerührt. Danach wurde eine Lösung von 3,6 g (0,025 mol) 4-Formylbenzoesäuremethylester und 1 g 3-Benzyl-5-(2-hydroxyethyl)-4-methylthiazoliumchlorid in 10 ml Dimethylformamid zugetropft. Man ließ 1 h nachrühren und goß danach auf Eis/Wasser, extrahierte 2 x mit Essigester, wusch die vereinigten organischen Extrakte mehrmals mit Wasser, trocknete über Magnesiumsulfat und engte ein. Aus dem Rückstand (6,4 g) erhielt man nach Umkristallisation aus Ethanol 3,7 g der Titelverbindung, Schmp. 139-141°C.

### Beispiel 6

### 1-(4-Carbomethoxyphenyl-2-(5,6,7,8-tetrahydro-5,5,8,8-tetramethyl-2-naphthalenyl)ethandion

Zu einer Suspension von 34,5 g (66 mmol) (4-carbomethoxyphenyl)methyl-triphenylphosphoniumbromid in 200 ml trockenem Toluol gab man bei Raumtemp. portionsweise 7,9 g (33 mmol) Kalium-tert.-butanolat zu. Man ließ 10 min nachrühren, erhitzte danach zum Rückfluß und tropfte 7,5 g (33 mmol) 5,6,7,8-Tetrahydro-5,5,8,8-tetramethylnaphthalin-2-carbonsäurechlorid zu. Nachdem die Lösung sich wieder entfärbt hatte, ließ man abkühlen und filtrierte vom Feststoff ab. Das Filtrat wurde eingeengt und das verbleibende Öl aus Methanol umkristallisiert (11,2 g der Ylid-Zwischenstufe, Schmp. 201-203°C). 3,2 g dieses Ylids und 3,1 g Natriumperiodat wurden in einem Gemisch aus 40 ml Ethanol und 10 ml Wasser 1 h unter Rückfluß erhitzt. Nach dem Abkühlen wurde das Reaktionsgemisch mit ges. Natriumchloridlösung versetzt und mit Methylenchlorid extrahiert. Die organische Phase wurde über Magnesiumsulfat getrocknet und eingeengt. Der Rückstand wurde in n-Heptan verrührt, man filtrierte Feststoffe ab und engte das Filtrat ein. Nach Umkristallisation aus Ethanol erhielt man 1,5 g der Titelverbindung, Schmp. 93-96°C.

### Beispiel 7

### 2-(4-Carboxyphenyl)-1-(5,6,7,8-tetrahydro-3-methoxy-5,5,8,8-tetramethyl-2-naphthalenyl)ethanon

14,6 g (29 mmol) 1,2-Dibrom-1-(4-cyanophenyl)-2-(5,6,7,8-tetrahydro-3-methoxy-5,5,8,8-tetramethyl-2-naphthalenyl)ethan, das durch Bromierung von (E)-4-[2-(5,6,7,8-tetrahydro-3-methoxy-5,5,8,8-tetramethyl-2-naphthalenyl)-1-ethenyl]benzonitril mit elementarem Brom in Chloroform erhalten worden war, und 15,3 g Kaliumhydroxidplätzchen wurden in 38 ml n-Butanol 1 h unter Rückfluß erhitzt. Danach goß man auf Eis/Wasser, säuerte mit 2 N Salzsäure an und extrahierte 3 x mit Ether. Die vereinigten Etherextrakte wurden mit Wasser neutral gewaschen, über Natriumsulfat getrocknet und eingeengt. Nach 3 Tagen war in dem n-Butanol-haltigen Rückstand ein Kristallisat entstanden, das abgesaugt und mit wenig Ethanol gewaschen wurde. Man erhielt so 2,9 g der Titelverbindung, Schmp. 164-165°C.

### Beispiel 8

### 2-(4-Carbomethoxyphenyl)-1-(5,6,7,8-tetrahydro-5,5,8,8-tetra-methyl-2-naphthalenyl)ethanon

Zu einer Lösung bzw. Suspension von 0,56 g Bis(triphenylphosphin)-palladium(II)-dichlorid und 2,1 g Zinkpulver in 40 ml Dimethoxyethan wurde bei Raumtemp. eine Lösung von 4 g (16 mmol) 5,6,7,8-Tetrahydro-5,5,8,8-tetramethylnaphthalin-2-carbonsäure-chlorid und 4 g (16 mmol) 4-(Brommethyl)benzoesäuremethylester in 20 ml Dimethoxyethan zugetropft. Danach wurde das Reaktionsgemisch 6 h unter Rückfluß erhitzt. Man ließ abkühlen, filtrierte vom Feststoff ab, erhitzte das Filtrat mit Aktivkohle und filtrierte heiß ab. Das Filtrat wurde eingeengt und ergab nach wiederholter Umkristallisation 3 x aus Methanol und schließlich aus Isopropanol, 0,9 g der Titelverbindung, Schmp. 103-104°C.

### Beispiel 9

### 2-(4-Carbomethoxyphenyl)-1-(5,6,7,8-tetrahydro-4-methoxy-5,5,8,8-tetramethyl-2-napthalenyl)ethanon

5,6,7,8-Tetrahydro-4-methoxy-5,5,8,8-tetramethylnaphthalin-2-carbonsäurechlorid:
20 g (86 mmol) 1,2,3,4-Tetrahydro-5-methoxy-1,1,4,4,7-pentamethylnaphthalin, 4,9 g Natriumhydroxid, 58 ml Pyridin und 30 ml Wasser wurden zusammengegeben und auf 95°C erhitzt. Bei dieser Temperatur wurden 32,5 g Kaliumpermanganat portionsweise zugegeben. Man rührte 2 h bei 95°C nach, ließ abkühlen und tropfte 6 ml Ethanol langsam zu. Anderntags wurde der Braunstein abfiltriert und mit heißer 2 N Natronlauge nachgewaschen. Das Filtrat (2 Phasen) wurde mit Ether versetzt, wonach 3 Phasen vorlagen. Die mittlere Phase wurde abgetrennt, 2 x mit Petrolether extrahiert und mit konz. Salzsäure angesäuert. Der ausgefallene weiße Niederschlag wurde abgesaugt und getrocknet: 9,2 g 5,6,7,8-Tetrahydro-4-methoxy-5,5,8,8-tetramethylnaphthalin-2-carbonsäure.

5 g (19 mmol) davon wurden in 30 ml Toluol gelöst. Man gab 3 Tropfen Dimethylformamid zu und tropfte dann 2,9 g (23 mmol) Oxalsäurechlorid langsam zu. Man ließ 30 min bei Raumtemp. nachrühren, erwärmte anschließend auf 70°C und rührte bei dieser Temperatur, bis keine Gasentwicklung mehr zu beobachten war. Man ließ abkühlen und entfernte das Lösungsmittel im Vakuum. Zurück blieben 6,1 g rohes 5,6,7,8-Tetrahydro-4-methoxy-5,5,8,8-tetramethylnaphthalin-2-carbonsäurechlorid, dessen Struktur durch H-NMR abgesichert wurde.

### 2-(4-Carbomethoxyphenyl)-1-(5,6,7,8-tetrahydro-4-methoxy-5,5,8,8-tetra-methyl-2-napthalenyl)ethanon

2,1 g Zinkpulver und 0,21 g Kupfer(II)-acetat wurde unter Kühlung mit 7,3 ml Essigsäure 30 min bei 20°C gerührt. Das Zink-Kupfer-Paar wurde abgesaugt, 2 x mit trockenem Ether und 1 x mit trockenem Dimethoxyethan gewaschen. Zu einer Suspension aus dem solchermaßen hergestellten Zink-Kupfer-Paar und 0,56 g Bis(triphenylphosphin)palladium(II)-chlorid in 15 ml Dimethoxyethan wurde eine Lösung von 4,5 g (16 mmol) 5,6,7,8-Tetrahydro-4-methoxy-5,5,8,8-tetramethylnaphthalin-2-carbonsäurechlorid und 3,7 g (16 mmol) 4-(Brommethyl)benzoesäuremethylester in 60 ml Dimethoxyethan bei Raumtemp. zugetropft. Man ließ 75 min rühren. Anschließend wurde das Reaktionsgemisch über Celite filtriert und das Filtrat eingeengt. Der Rückstand ergab nach dreimaliger Umkristallisation aus Methanol 2,0 g der Titelverbindung, Schmp. 143-144°C.

### Beispiel 10

### 1-(4-Carbomethoxyphenyl)-2-(5,6,7,8-tetrahydro-5,5,8,8-tetra-methyl-2-naphthalenyl)ethanon

6-(Brommethyl)-1,2,3,4-tetrahydro-1,1,4,4-tetramethylnaphthal in: Zu einer Lösung von 121 g (0,6 mol) 1,2,3,4-Tetrahydro-1,1,4,4,6-pentamethylnaphthalin und 1 Spatelspitze 2,2′-Azodiisobutyronitril in 1 l 1,2-Dichlorethan gab man bei 80°C portionsweise ein Gemisch aus 117,5 g (0,66 mol) N-Bromsuccinimid und 2 g 2,2′-Azodiisobutyronitril zu. Nach Beendigung der Zugabe wurde 75 min bei ca. 80°C nachgerührt. Man ließ danach abkühlen, zog das Lösungsmittel im Vakuum ab und verrührte den Rückstand mit n-Heptan. Der Niederschlag wurde abfiltriert und das Filtrat eingeengt. Der Rückstand wurde destilliert, und man erhielt so 109 g 6-(Brommethyl)-1,2,3,4-tetrahydro-1,1,4,4-tetramethylnaphthalin, Sdp. 122-124°C (0,3 mbar).

### 1-(4-Carbomethoxyphenyl)-2-(5,6,7,8-tetrahydro-5,5,8,8-tetra-methyl-2-naphthalenyl)ethanon:

Analog Beispiel 9 erhielt man aus 10 g (50 mmol) Terephthalsäuremonomethylesterchlorid und 14 g (50 mmol) 6-(Brommethyl)-1,2,3,4-tetrahydro-1,1,4,4-tetramethylnaphthalin 0,3 g der Titelverbindung, Schmp. 96-97°C.

### Beispiel 11

### 2-(4-Carboxyphenyl)-1-(5,6,7,8-tetrahydro-5,5,8,8-tetramethyl-2-naphthalenyl)ethanon

65,4 g (0,3 mol) 5,6,7,8-Tetrahydro-5,5,8,8-tetramethyl-2-naphthaldehyd wurden mit 56 g (0,38 mol) Orthoameisensäureethylester und 0,3 g 4-Toluolsulfonsäure in 75 ml absolutem Ethanol 16 h bei Raumtemp. gerührt. Danach gab man 7,6 g wasserfreies Natriumcarbonat zu, rührte ca. 15 min, filtrierte den Feststoff ab und engte das Fitrat ein. Es verbleiben 92 g Diethylacetal. Zu einer Lösung von 73,5 g (0,25 mol) dieses Diethylacetals und 42,2 g (0,25 mol) Triethylphosphit in 240 ml trockenem Methylenchlorid wurden bei -20°C unter Stickstoff 38 g (0,265 mol) Bortrifluorid-diethyletherat getropft. Man ließ über Nacht langsam auf Raumtemp. kommen, versetzte dann mit 80 ml Wasser, trennte die Phasen, trocknete die organische Phase über Natriumsulfat und engte ein. Nach säulenchromatographischer Reinigung (Kieselgel; n-Heptan/Essigester 1:0 → 1) erhielt man 33,3 g leicht verunreinigten 1-Ethoxy-1-(5,6,7,8-tetrahydro-5,5,8,8-tetramethyl-2-naptha-lenyl)methyl-phosphonsäurediethylester als Öl dessen Struktur durch H- und ¹³C-NMR abgesichert wurde.

Zu einer Lösung von 7,6 g (20 mmol) dieses Phosphonats in 30 ml absolutem Tetrahydrofuran wurden bei -78°C unter Stickstoff 28 ml (44 mmol) einer 1,6 M Lösung von n-Butyllithium in n-Hexan langsam zugetropft. Man ließ 30 min nachrühren und tropfte dann ebenfalls bei -78°C eine Lösung von 3,3 g (20 mmol) 4-formylbenzoesäuremethylester in 20 ml Tetrahydrofuran zu. Man ließ auf Raumtemp. kommen und rührte noch 1 h nach. Das Reaktionsgemisch wurde auf Wasser gegeben, mit Ether extrahiert, die Etherphase mit Wasser gewaschen, über Natriumsulfat getrocknet und eingeengt. Der Rückstand wurde säulenchromatographisch (Kieselgel; n-Heptan/Essigester 95:5) grob gereinigt, und man erhielt 6,7 g noch schwach verunreinigtes 2-(4-Carbomethoxyphenyl)-1-ethoxy-1-(5,6,7,8-tetrahydro-5,5,8,8-tetramethyl-2-napthalenyl)ethen. 2 g (5 mmol) davon wurden mit 0,5 g Kaliumhydroxid in einem Gemisch aus 15 ml Methanol und 10 ml Wasser 3 h unter Rückfluß erhitzt. Nach dem Abkühlen wurde auf Wasser gegossen und mit Ether extrahiert. Die wäßrige Phase wurde mit 2 N Salzsäure angesäuert, und das sich ölig abscheidende Rohprodukt mit Ether extrahiert. Der Etherextrakt wurde eingeengt und der Rückstand in 16 ml Tetrahydrofuran gelöst. Man setzte 4 ml 2 N Salzsäure zu und erhitzte das Reaktionsgemisch 3 h unter Rückfluß. Man ließ abkühlen, gab Wasser zu, trennte die Phasen, und engte die organische Phase ein. Als Rückstand erhielt man 0,5 g der Titelverbindung, Schmp. 204-206°C, R_{F}=0,39 (DC:Kieselgel; Methylenchlorid/Methanol 9:1).

### Beispiel 12

### 1-(4-Carboxyphenyl)-2-(5,6,7,8-tetrahydro-5,5,8,8-tetramethyl-2-naphthalenyl)ethandion

3,9 g (10 mmol) 1-(4-carbomethoxyphenyl)-2-(5,6,7,8-tetrahydro-5,5,8,8-tetramethyl-2-naphthalenyl)ethandion (Beispiel 6) wurden in einem Gemisch aus 5 ml konz. Schwefelsäure, 80 ml Eisessig und 40 ml Wasser 8 h unter Rückfluß erhitzt. Danach goß man in 500 ml Wasser, saugte das ausgefallene Kristallisat ab und wusch es mit Wasser bis zu neutralem pH des Waschwassers. Das getrocknete Rohprodukt ergab nach Umkristallisation aus Cyclohexan 3,1 g der Titelverbindung, Schmp. 148-151°C.

## Patentansprüche

1. Verbindungen der allgemeinen Formel I 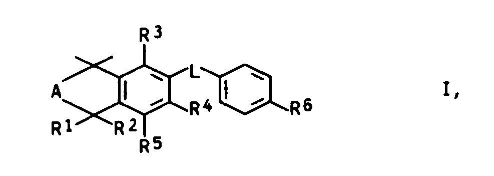 in der bedeuten:
A einen gegebenenfalls mit einer Methyl-, Hydroxyl- oder Oxogruppe substituierten Ethylen- oder Methylenrest,
L eine der Gruppen 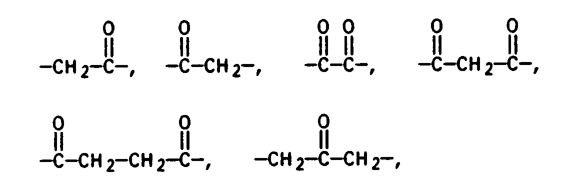 R¹ und R² Wasserstoff oder einen Methylrest,
R³ Wasserstoff, eine Hydroxy- oder eine C₁₋₆-Alkoxygruppe,
R⁴ Wasserstoff, einen C₁₋₄-Alkylrest, ein Halogenatom oder eine Methoxygruppe,
R⁵ Wasserstoff oder eine Methoxygruppe und
R⁶ Wasserstoff, eine Methyl- oder Nitrilgruppe, eine C₂₋₁₀-Ketalgruppe oder die Reste
-CHR⁷-OR⁸, -CHR⁷-NR⁹R¹⁰, -COR¹¹, 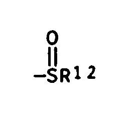 oder -SO₂R¹¹,
worin bedeuten:
R⁷ Wasserstoff oder eine C₁₋₄-Alkylgruppe,
R⁸ Wasserstoff, eine C₁₋₄-Alkyl-, C₁₋₂₀-Alkanoyl- oder eine gegebenenfalls substituierte Benzoylgruppe,
R⁹ und R¹⁰ Wasserstoffatome, C₁₋₄-Alkyl-, C₁₋₆-Alkanoyl- oder gegebenenfalls substituierte Benzoylgruppen oder zusammen mit dem Stickstoffatom, an das sie gebunden sind, einen 5- oder 6-gliedrigen, gesättigten heterocyclischen Rest, der außer dem Stickstoffatom auch zusätzlich ein Sauerstoffatom enthalten kann,
R¹¹ Wasserstoff, eine C₁₋₄-Alkylgruppe oder die Reste -OR¹³ oder -NR¹⁴R¹⁵ mit R¹³ in der Bedeutung eines Wasserstoffatoms, einer gegebenenfalls durch 1 oder 2 Hydroxylgruppen substituierten C₁₋₈-Alkylgruppe, einer gegebenenfalls substituierten Aryl- oder gegebenenfalls im Arylteil substituierten Aralkylgruppe, und mit R¹⁴ und R¹⁵ in der Bedeutung von Wasserstoffatomen, gegebenenfalls substituierten Aralkyl- oder Arylgruppen, oder R¹⁴ und R¹⁵ zusammen mit dem Stickstoffatom, an das sie gebunden sind, in der Bedeutung eines 5- oder 6-gliedrigen gesättigten heterocyclischen Restes, der außer dem Stickstoffatom auch zusätzlich ein Sauerstoffatom enthalten kann, und
R¹² eine C₁₋₄-Alkylgruppe,
sowie deren physiologisch verträgliche Salze.

2. 1-(4-Carbomethoxyphenyl)-3-(5,6,7,8-tetrahydro-5,5,8,8-tetramethyl-2-naphthalenyl)-1,3-propandion.

3. 1-(4-Carboxyphenyl)-3-(5,6,7,8-tetrahydro-5,5,8,8-tetramethyl-2-naphthalenyl)-1,3-propandion.

4. 2-(4-Carbomethoxyphenyl)-1-(5,6,7,8-tetrahydro-4-methoxy-5,5,8,8-tetramethyl-2-naphthalenyl)ethanon.

5. Verfahren zur Herstellung von Desoxybenzoinen der Formel I gemäß einem der Ansprüche 1 bis 4 (L = -CH₂-CO- oder -CO-CH₂-), dadurch gekennzeichnet, daß man
a) einen Phosphonsäureester der Formel II 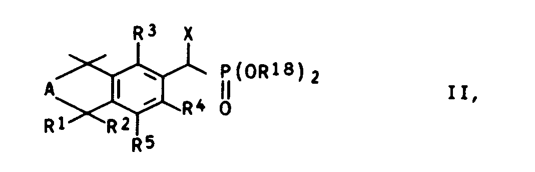 worin A und R¹ bis R⁵ die im Anspruch 1 genannten Bedeutungen haben und X einen Rest -OR¹⁶ oder NR¹⁶R¹⁷ bedeutet, wobei die Reste R¹⁶, R¹⁷ und R¹⁸ jeweils einen C₁₋₄-Alkylrest bedeuten,
mit einem Aldehyd der Formel III 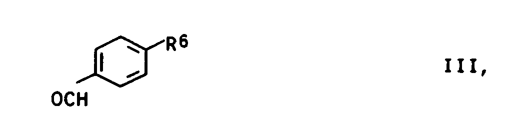 in der R⁶ die im Anspruch 1 genannte Bedeutung hat,
in Gegenwart einer Base im Sinne einer Wittig-Horner-Reaktion umsetzt und das daraus erhaltene Enamin (X = NR¹⁶R¹⁷) bzw. den Enolether (X = OR¹⁶) sauer hydrolysiert, oder daß man
b) ein Carbonsäurechlorid der Formel IV oder V 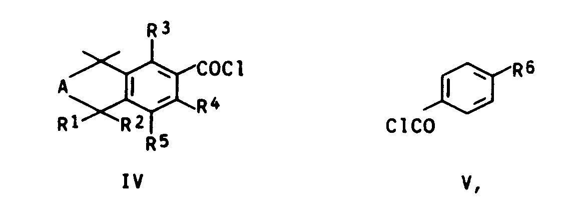 in denen A und R¹ bis R⁶ die im Anspruch 1 genannten Bedeutungen haben, mit einem Benzylhalogenid der Formel VI oder VII 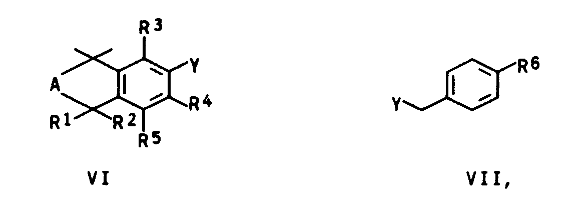 in denen A und R¹ bis R⁶ die im Anspruch 1 genannten Bedeutungen haben und Y für Chlor oder Brom steht,
in Gegenwart eines geeigneten Metalls, gegebenenfalls unter Zusatz katalytischer Mengen eines Übergangsmetallkatalysators, umsetzt, wobei entweder IV mit VII oder V mit VI umgesetzt wird.

6. Verfahren zur Herstellung von 1,3-Diketoverbindungen der Formel I gemäß einem der Ansprüche 1 bis 4 (L = -C(O)CH₂C(O)-) oder deren enoltautomeren Verbindungen, dadurch gekennzeichnet, daß man
a) ein Keton der Formel VIII 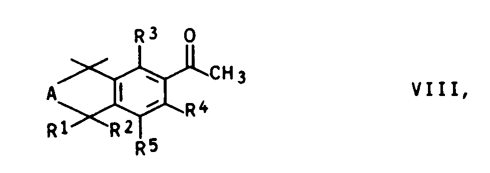 in der A und R¹ bis R⁵ die im Anspruch 1 genannten Bedeutungen haben, mit einem Terephthalsäureester der Formel IX 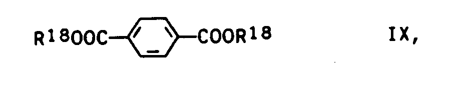 in der R¹⁸ für eine C₁₋₄-Alkylgruppe steht,
b) in Gegenwart einer Base umsetzt, oder indem man
an ein Chalkon der Formel X 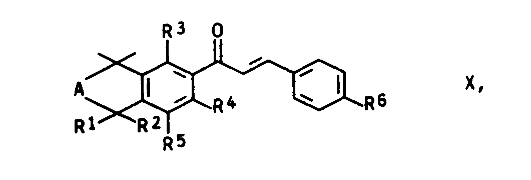 in der A und R¹ bis R⁶ die im Anspruch 1 genannten Bedeutungen haben,
zunächst Brom addiert, dann mit einer geeigneten Base Bromwasserstoff eliminiert und schließlich sauer hydrolysiert.

7. Verfahren zur Herstellung von 1,4-Diketoverbindungen der Formel I gemäß einem der Ansprüche 1 bis 4 (L = -C(O)CH₂CH₂C(O)-), dadurch gekennzeichnet, daß man ein Chlorethylketon der Formel XI 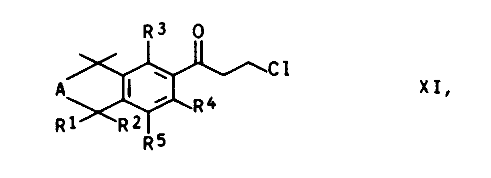 in der A und R¹ bis R⁵ die im Anspruch 1 genannten Bedeutungen haben, mit einer Base zunächst zu einem Vinylketon der Formel XII 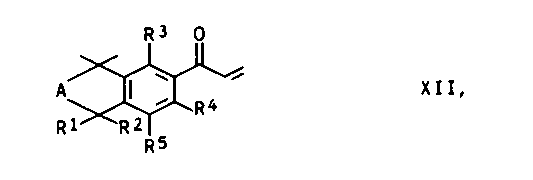 in der A und R¹ bis R⁵ die im Anspruch 1 genannten Bedeutungen haben, umsetzt, und dieses mit einem Benzaldehyd der Formel XIII 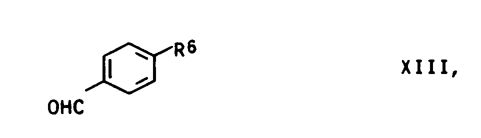 in der R⁶ die im Anspruch 1 genannte Bedeutung hat,
in Gegenwart eines "Umpolungs"-Katalysators in an sich bekannter Weise weiter umsetzt.

8. Kosmetika und Arzneimittel zur topischen Anwendung, die neben üblichen galenischen Hilfsstoffen 0,0001 bis 1 Gew.% einer Verbindung der allgemeinen Formel I nach einem der Ansprüche 1 bis 4 als Wirkstoff enthalten.

9. Arzneimittel zur systemischen Anwendung, das neben üblichen galenischen Hilfsstoffen pro Einzeldosis 0,1 bis 50 mg einer Verbindung der allgemeinen Formel I nach Anspruch 1 als Wirkstoff enthält.

10. Arzneimittel nach Anspruch 8 oder 9 zur Bekämpfung von Akne, Psoriasis und anderen, mit pathologisch veränderter Verhornung einhergehenden dermatologischen Erkrankungen und durch UV-Licht ausgelösten oder iatrogen bedingten Hautschädigungen sowie zur Behandlung von Ekzemen, Warzen, Vitiligo, ferner Präkanzerosen und Tumoren, trockenen Augen und anderen Corneopathien sowie rheumatischen und arthritischen Erkrankungen.

## Claims

1. A compound of the formula I 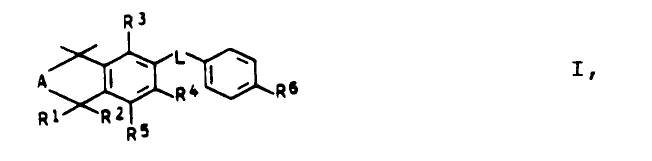 where
A is ethylene or methylene which can be substituted by methyl, hydroxyl or oxo,
L is one of the following 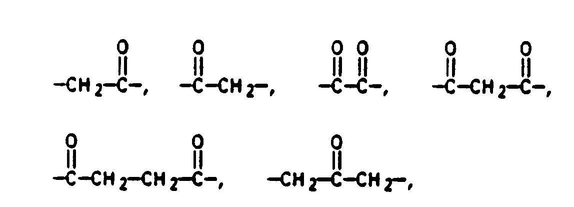 R¹ and R² are hydrogen or methyl,
R³ is hydrogen, hydroxyl or C₁-C₆-alkoxy,
R⁴ is hydrogen, C₁-C₄-alkyl, halogen or methoxy,
R⁵ is hydrogen or methoxy, and
R⁶ is hydrogen, methyl, cyano, a C₂-C₁₀ group of the formula 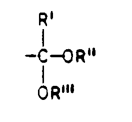 where R',R" and R'" are each alkyl of 1 to 9 carbons, the total of carbons in R',R",R'" is 2 to 10, and R' can also be hydrogen, or the following
-CHR⁷-OR⁸, -CHR⁷-NR⁹R¹⁰, -COR¹¹, 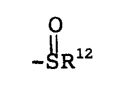 or -SO₂R¹¹
where
R⁷ is hydrogen or C₁-C₄-alkyl,
R⁸ is hydrogen, C₁-C₄-alkyl, C₁-C₂₀-alkanoyl, or benzoyl which can be substituted,
R⁹ and R¹⁰ are hydrogen, C₁-C₄-alkyl, C₁-C₆-alkanoyl, or benzoyl which can be substituted, or form, together with the nitrogen to which they are bonded, a 5- or 6-membered saturated heterocyclic radical which can also contain an oxygen in addition to the nitrogen,
R¹¹ is hydrogen, C₁-C₄-alkyl, or -OR¹³ or -NR¹⁴R¹⁵ where R¹³ is hydrogen, C₁-C₈-alkyl which can be substituted by 1 or 2 hydroxyls, or aryl or aralkyl each of which can be substituted in the aryl moiety, and where R¹⁴ and R¹⁵ are hydrogen, or aralkyl or aryl which can be substituted, or R¹⁴ and R¹⁵ form, together with the nitrogen to which they are bonded, a 5- or 6-membered saturated heterocyclic radical which can also contain an oxygen in addition to the nitrogen, and
R¹² is C₁-C₄-alkyl,
and the physiologically tolerated salts thereof.

2. 1-(4-Carbomethoxyphenyl)-3-(5,6,7,8-tetrahydro-5,5,8,8,-tetramethyl-2-naphthyl)-1,3-propanedione.

3. 1-(4-Carboxyphenyl)-3-(5,6,7,8-tetrahydro-5,5,8,8-tetramethyl-2-naphthyl)-1,3-propanedione.

4. 2-(4-Carbomethoxyphenyl)-1-(5,6,7,8-tetrahydro-4-methoxy-5,5,8,8-tetramethyl-2-naphthyl)ethanone.

5. A process for the preparation of a deoxybenzoin of the formula I as claimed in any of claims 1 to 4 (L = -CH₂-CO- or -CO-CH₂-), which comprises
a) reacting a phosphonic ester of the formula II 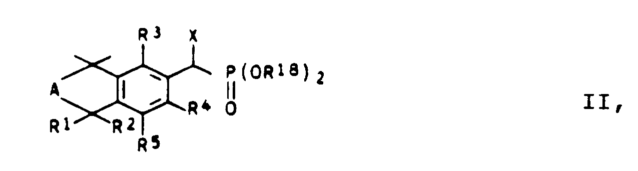 where A and R¹-R⁵ have the meanings specified in claim 1, and X is -OR¹⁶ or NR¹⁶R¹⁷ where R¹⁶, R¹⁷ and R¹⁸ are each C₁-C₄-alkcyl, with an aldehyde of the formula III 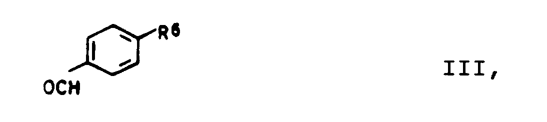 where R⁶ has the meanings specified in claim 1, in the presence of a base in a Wittig-Horner reaction, and subjecting the resulting enamine (X = NR¹⁶R¹⁷) or enol ether (X = OR¹⁶) to acid hydrolysis, or by
b) reacting a carbonyl chloride of the formula IV or V 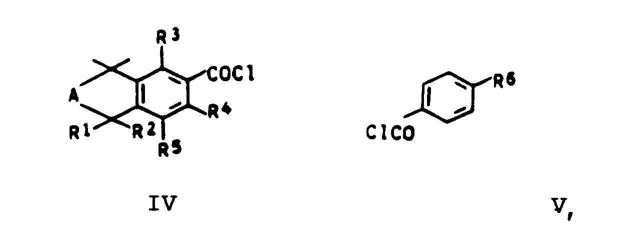 where A and R¹-R⁶ have the meanings specified in claim 1, with a benzyl halide of the formula VI or VII 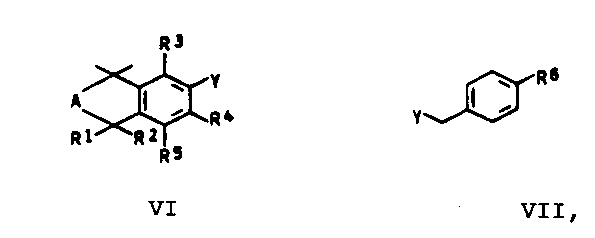 where A and R¹-R⁶ have the meanings specified in claim 1, and Y is chlorine or bromine, in the presence of a suitable metal, with or without the addition of catalytic amounts of a transition metal catalyst, the reaction being between either IV and VII or V and VI.

6. A process for the preparation of a 1,3-diketo compound of the formula I as claimed in any of claims 1 to 4 (L = -C(O)CH₂C(O)-) or of the enol tautomer thereof, which comprises
a) reacting a ketone of the formula VIII 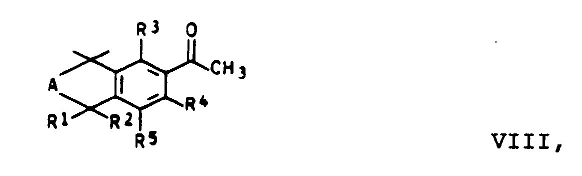 where A and R¹-R⁵ have the meanings specified in claim 1, with a terephthalic ester of the formula IX 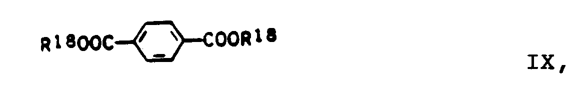 where R¹⁸ is C₁-C₄-alkyl, in the presence of a base, or by
b) subjecting a chalcone of the formula X 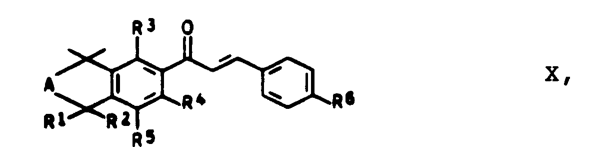 where A and R¹-R⁶ have the meanings specified in claim 1, to addition of bromine, then eliminating hydrogen bromide with a suitable base, and finally carrying out acid hydrolysis.

7. A process for the preparation of a 1,4-diketo compound of the formula I as claimed in any of claims 1 to 4, (L = -C(O)CH₂CH₂C(O)-), which comprises reacting a chloroethyl ketone of the formula XI 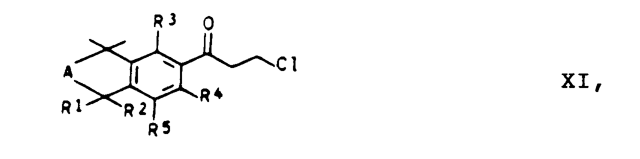 where A and R¹ to R⁵ have the meanings specified in claim 1, with a base to give a vinyl ketone of the formula XII 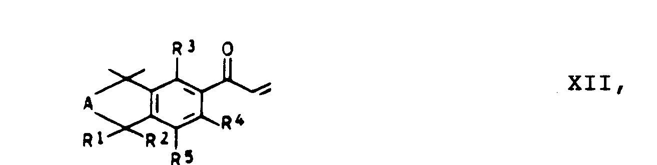 where A and R¹ to R⁵ have the meanings specified in claim 1, and then reacting the latter with a benzaldehyde of the formula XIII 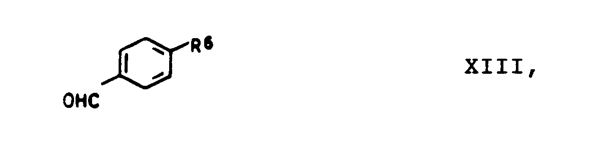 where R⁶ has the meaning specified in claim 1, in the presence of an "umpolung" catalyst in a conventional manner.

8. A cosmetic or drug for topical use, which contains 0.0001 to 1% by weight of a compound of the formula I as claimed in any of claims 1 to 4 as active substance, in addition to conventional pharmaceutical auxiliaries.

9. A drug for systemic use, which contains 0.1 to 50 mg of a compound of the formula I as claimed in claim 1 as active substance per single dose, in addition to conventional pharmaceutical auxiliaries.

10. A drug as claimed in claim 8 or 9 for controlling acne, psoriasis and other dermatological disorders associated with pathological keratinization, and UV-induced or iatrogenic skin damage and for the treatment of eczema, warts, vitiligo as well as precanceroses and tumors, dry eyes and other corneopathies as well as rheumatic and arthritic disorders.

## Revendications

1. Composés de la formule générale I 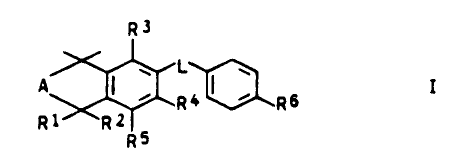 dans laquelle
A représente un radical méthylène ou éthylène, éventuellement substitué par un groupe méthyle, hydroxyle ou oxo,
L représente l'un des radicaux qui suivent 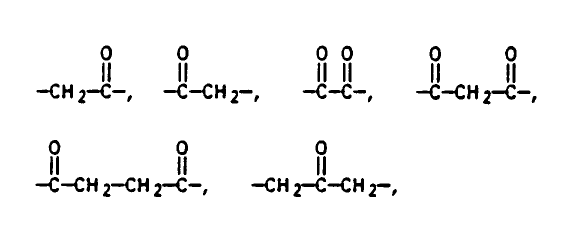 R¹ et R² représentent chacun un atome d'hydrogène ou le radical méthyle,
R³ représente un atome d'hydrogène, le radical hydroxyle ou un radical alcoxy en C₁-C₆,
R⁴ représente un atome d'hydrogène, un radical alkyle en C₁-C₄, un atome d'halogène, ou le radical méthoxy,
R⁵ représente un atome d'hydrogène ou le radical méthoxy et
R⁶ représente un atome d'hydrogène, le radical méthyle ou nitrile, un radical cétal en C₂-C₁₀, ou les radicaux
-CHR⁷-OR⁸, -CHR⁷-NR⁹R¹⁰, -COR¹¹, 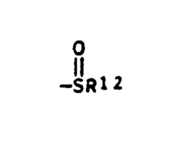 ou -SO₂R¹¹,
dans lesquels
R⁷ représente un atome d'hydrogène ou un radical alkyle en C₁-C₄,
R⁸ représente un atome d'hydrogène, un radical alkyle en C₁-C₄, alcanoyle en C₁-C₂₀, ou un radical benzoyle éventuellement substitué,
R⁹ et R¹⁰ représentent chacun un atome d'hydrogène, un radical alkyle en C₁-C₄, alcanoyle en C₁-C₆, ou un radical benzoyle éventuellement substitué, ou bien R⁹ et R¹⁰ forment, avec l'atome d'azote auquel ils sont liés, un radical hétérocyclique saturé, pentagonal ou hexagonal, qui, outre l'atome d'azote, peut complémentairement aussi contenir un atome d'oxygène,
R¹¹ représente un atome d'hydrogène, un radical alkyle en C₁-C₄, des radicaux -OR¹³ ou -NR¹⁴R¹⁵, dans lesquels R¹³ représente un atome d'hydrogène, un radical alkyle en C₁-C₈, éventuellement substitué par un ou deux groupes hydroxyle, un radical aryle éventuellement substitué ou un radical aralkyle à groupe aryle éventuellement substitué et R¹⁴ et R¹⁵ representent chacun un atome d'hydrogène, un radical aryle ou aralkyle, éventuellement substitué, ou bien R¹⁴ et R¹⁵ forment, avec l'atome d'azote auquel ils sont attachés, un radical hétérocyclique saturé, pentagonal ou hexagonal, qui, outre l'atome d'azote, peut complémentairement aussi contenir un atome d'oxygène et
R¹² représente un radical alkyle en C₁-C₄,
comme aussi leurs sels physiologiquement compatibles.

2. 1-(4-Carbométhoxyphényl)-3-(5,6,7,8-tétrahydro-5,5,8,8-tétraméthyl-2-naphtalényl)-1,3-propanedione.

3. 1-(4-Carboxyphényl)-3-(5,6,7,8-tétrahydro-5,5,8,8-tétraméthyl-2-naphtalényl)-1,3-propanedione.

4. 2-(4-Carbométhoxyphényl)-1-(5,6,7,8-tétrahydro-4-méthoxy-5,5,8,8-tétraméthyl-2-naphtalényl)éthanone.

5. Procédé de préparation de désoxybenzoïnes de la formule I suivant les revendications 1 à 4 (L = -CH₂CO-ou -CO-CH₂-), caractérisé en ce que
a) on fait réagir un ester de l'acide phosphonique de la formule II 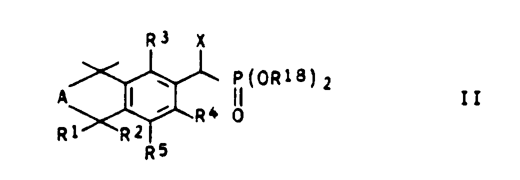 dans laquelle A et R¹ à R⁵ possèdent les significations qui leur ont été attribuées dans la revendication 1 et X représente un radical -OR¹⁶ ou NR¹⁶R¹⁷, où les symboles R¹⁶, R¹⁷ et R¹⁸ représentent chacun un radical alkyle en C₁-C₄,
avec un aldéhyde de la formule III 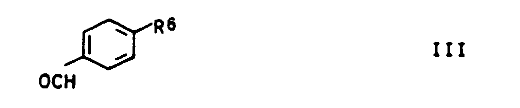 dans laquelle R⁶ possèdent les significations qui lui ont été attribuées dans la revendication 1,
en présence d'une base, à la manière d'une réaction de Wittig-Horner et on procède à l'hydrolyse acide de l'énamine qui en est obtenue (X = NR¹⁶R¹⁷) ou de l'éther ènolique (X = OR¹⁶), ou en ce que
b) on fait réagir un chlorure d'acide carboxylique de la formule IV ou V 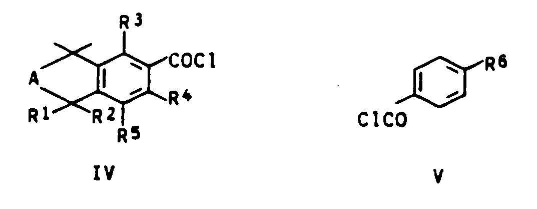 dans lesquelles A et R¹ à R⁶ possèdent les significations qui leur ont été attribuées dans la revendication 1, avec un halogénure de benzyle de la formule VI ou VII 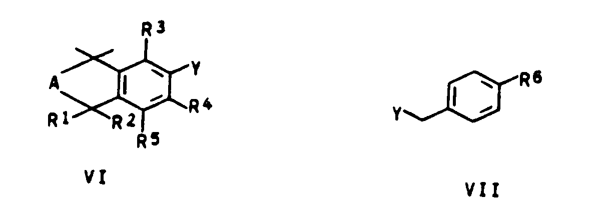 dans lesquelles A et R¹ à R⁶ possèdent les significations qui leur ont été attribuées dans la revendication 1 et Y représente un atome de chlore ou de brome,
en présence d'un métal approprié, éventuellement sous addition de proportions catalytiques d'un catalyseur à métal de transition, où l'on fait réagir le composé IV avec le composé VII ou le composé V avec le composé VI.

6. Procédé de préparation de composés du type 1,3-dicéto de la formule I selon l'une quelconque des revendications 1 à 4 (L = -C(O)CH₂C(O)-), ou de leurs tautomères énoliques, caractérisé en ce que
a) on fait réagir une cétone de la formule VIII 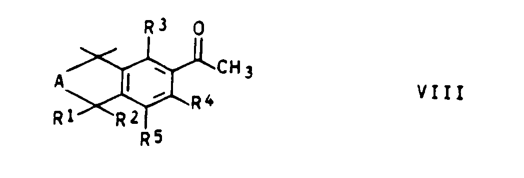 dans laquelle A et R¹ à R⁵ possèdent les significations qui leur ont été attribuées dans la revendication 1, avec un ester de l'acide téréphtalique de la formule IX 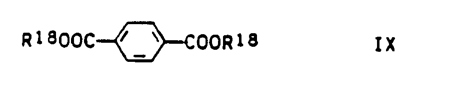 dans laquelle R¹⁸ représente un radical alkyle en C₁-C₄,
b) en présence d'une base, ou en ce que
on additionne d'abord du brome sur une chalcone de la formule X 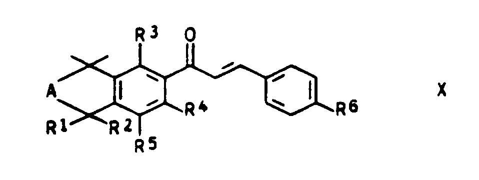 dans laquelle A et R¹ à R⁶ possèdent les significations qui leur ont été attribuées dans la revendication 1,
puis on élimine de l'acide bromhydrique avec une base appropriée et on procède finalement à une hydrolyse acide.

7. Procédé de préparation de composés du type 1,4-dicéto de la formule I selon l'une quelconque des revendications 1 à 4 (L = -C(O)CH₂CH₂C(O)-), caractérisé en ce que l'on convertit d'abord une chloréthylcétone de la formule XI 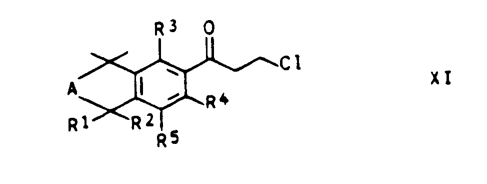 dans laquelle A et R¹ à R⁵ possèdent les significations qui leur ont été attribuées dans la revendication 1, à l'aide d'une base en une vinylcétone de la formule XII 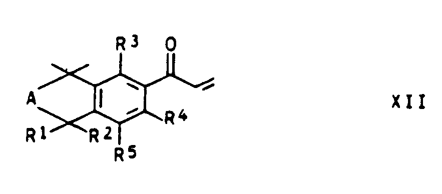 dans laquelle A et R¹ à R⁵ possèdent les significations qui leur ont été attribuées dans la revendication 1 et on fait davantage réagir cette dernière avec un benzaldéhyde de la formule XIII 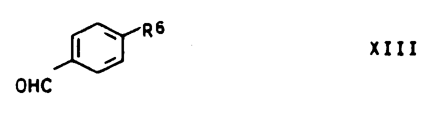 dans laquelle R⁶ possède les significations qui lui ont été attribuées dans la revendication 1,
en présence d'un catalyseur de "transpolation" de manière en soi connue.

8. Cosmétiques et médicaments destinés à l'utilisation topique, qui contiennent de 0,0001 à 1% en poids d'un composé de la formule générale I suivant l'une quelconque des revendications 1 à 4, à titre de principe actif, en plus des excipients ou adjuvants galéniques usuels.

9. Médicament destiné à l'utilisation systémique, qui contient de 0,1 à 50 mg d'un composé de la formule I suivant la revendication, à titre de principe actif, par dose unitaire, en plus des excipients ou adjuvants galéniques habituels.

10. Médicaments suivant la revendication 8 ou 9 pour lutter contre l'acné, le psoriasis et d'autres maladies dermatologiques associées à une kératinisation pathologiquement modifiée et contre des dommages cutanés déclenchés par la lumière ultraviolette ou à cause iatrogène, comme aussi pour le traitement d'eczémas, de verrues, du vitiligo, de tumeurs et d'états précancéreux, des yeux secs et d'autres cornéopathies, comme aussi de maladies rhumatismales et arthritiques.
